# EUROPEAN PATENT SPECIFICATION

(11) **EP 3 421 503 B1**
(45) Date of publication and mention of the grant of the patent: **20.05.2020**
(21) Application number: 16865579.3
(22) Date of filing: 19.08.2016
(51) Int. Cl.: C08B 37/16, A61K 31/724

(54) **SUGAMMADEX PREPARATION AND PURIFICATION METHOD**
SUGAMMADEX HERSTELLUNG UND REINIGUNGSVERFAHREN
PROCÉDÉ DE PRÉPARATION ET DE PURIFICATION DE SUGAMMADEX

(30) Priority: 29.06.2016 CN 201610498672
(43) Date of publication of application: 02.01.2019
(73) Proprietor: Beijing Creatron Institute Of Pharmaceutical Research Co., Ltd., Beijing 102200 (CN)
(72) Inventor: JIA, Huijuan, Beijing 102200 (CN); CHEN, Yan, Beijing 102200 (CN); LIU, Xiangwei, Beijing 102200 (CN)
(74) Representative: Hoffmann Eitle
(86) International application number: PCT/CN2016/095985
(87) International publication number: WO 2017/084401

(56) References cited:
- WO-A1-01/40316
- WO-A1-2012/025937
- WO-A1-2014/125501
- WO-A1-2014/125501
- CN-A- 1 402 737
- CN-A- 104 628 891
- CN-A- 104 844 732

## Description

### FIELD OF THE INVENTION

The present invention belongs to the field of drug synthesis and relates to the preparation of active pharmaceutical ingredients and intermediates. More particularly, the present invention relates to a process for the preparation and purification of Sugammadex sodium and its intermediates.

### BACKGROUND OF THE INVENTION

Sugammadex sodium (abbreviated as SGMD) was first discovered by Organon Biosciences. In 2007, Organon Biosciences was acquired by Schering-Plough, the latter merged with Merck & Co. in 2009. SGMD is now owned and sold by Merck & Co.. SGMD and its injection were approved by EMEA at the end of 2009, of which the tradename is Bridion. In 2010, SGMD was approved by PMDA and then by FDA in Dec. 2015. Since then, SGMD and its injection have been launched in more than 50 countries all over the world. In 2015, CFDA approved the application of SGMD injection as investigational new drug (IND) by N.V. Organon's.

Sugammadex sodium represents the first and only selective relaxant binding agent (SRBA) and is one of the most notable achievements in the field of anaesthetic during the last 20 years. SGMD chelates free rocuronium bromide molecules thus rapidly reduces the concentration thereof in plasma. The transfer of rocuronium bromide molecule from neuromuscular junction to plasma caused by the concentration difference between them renders in the decrease of the concentration of rocuronium bromide molecule at neuromuscular junction so that rocuronium bromide molecule bound to nicotinic acetylcholine receptors (nAChRs) is released at neuromuscular junction which results in the reversal of neuromuscular blockade induced by rocuronium bromide molecule.

Sugammadex sodium binds to and inactivates non-polarizing muscle relaxants with high selectivity. It antagonizes rocuronium bromide molecule due to the chelating between the lipophilic core of SGMD and the rocuronium bromide molecules. The similar antagonism works also to vecuronium bromide which is an analogue of rocuronium bromide molecule. However, no reversal effect for neither the non-depolarizing muscle relaxants with the structure of benzyl isoqunoline e.g., cis-atracurium, nor the depolarizing neuromulscular blocking agents e.g., succinylcholine.

Sugammadex sodium is a modified derivative of γ-cyclodextrin which contains 8 glucopyranose units with a lipophilic core and a hydrophilic periphery. The full chemical name of SGMD is 6-per-deoxy-6-per-(2-carboxyethyl)thio-γ-cyclodextrin, sodium salt. Its structure is shown as below:

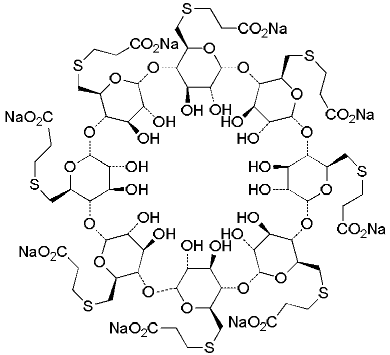

Due to its complex structure, extreme polarity and good water solubility, the preparation and purification of SGMD becomes very difficult since the impurities produced during the preparation thereof have physico-chemical characteristics and molecular weights comparable to that of the active substance. The current existing methods for the preparation of SGMD are as listed as follows:
1. Patent CN1188428C (cognate patent of EP1259550B1) assigned to Akzo Noble, the inventor of Sugammadex sodium.

The process described in this patent started from γ-cyclodextrin, which reacts with iodine and triphenylphosphine to afford an intermediate, 6-per-deoxy-6-iodo-γ-cyclodextrin (SGMD-1). The intermediate SGMD-1 thus prepared reacts with 3-mercaptopropionic acid to provide the crude Sugammadex sodium salt by nucleophilic substitution which was further purified by passing through macroporous resin and dialysis to remove impurities. The preparation of SGMD-1 according to this process requires cooling the reaction system prior to the addition of sodium methoxide and mixing the mixture prior to the addition of methanol and evaporation thereof. 2. Patents WO2012025937 and WO2014125501.

Compared to the route described in the patent CN1188428C, the process in this patent (WO2014125501) employed a different intermediate rather than SGMD-1. γ-cyclodextrin reacts with phosphorous pentachloride (or phosphorous pentabromide) instead of iodine and triphenylphosphine to afford 6-per-deoxy-6-chloro (or bromo)- γ-cyclodextrin which reacts with 3-mercaptopropionic acid by nucleophilic substitution to afford the crude SGMD which is further purified to obtain SGMD.

The preparation of SGMD described in patent WO2012025937 was same to that in patent WO2014125501, both of them use phosphorous pentahalide instead of iodine and triphenylphosphine used in CN1188428C. This process requires also the evaporation of DMF, a high boiling point organic solvent, at the end of the first step. In the second step, the addition of ethanol to the reaction system will precipitate the product of SGMD as well as the unreacted SGMD-1. Thus the purification requires silica gel and sephadex G25 column chromatography. While in patent WO2014125501, the crude SGMD was purified by recrystallization with methanol, ethanol, acetonitrile and water. Prior to recrystallization, active carbon was added to decolorize the product.

### DESCRIPTION OF THE INVENTION

The present invention relates to an industrially viable, cost effective process for the preparation of Sugammadex sodium.

In an embodiment of the present invention, the process described in this invention involves reacting γ-cyclodextrin (SM1) with iodine and triphenylphosphine in an organic solvent to afford an intermediate 1, 6-per-deoxy-6-iodo-γ-cyclodextrin (abbreviated as SGMD-1). After recrystallization, SGMD-1 was treated with 3-mercaptopropionic acid(SM2) in an organic solvent under basic condition to afford the crude 6-per-deoxy-6-per-(2-carboxyethyl)thio-γ-cyclodextrin, sodium salt (abbreviated as SGMD). The crude SGMD was further purified by absorbent and recrystallization.

In an embodiment of the present invention, the present invention provides a process for the preparation of Sugammadex sodium comprising:
reacting γ-cyclodextrin (SM1) with iodine in the presence of triphenylphosphine in an organic solvent to afford an intermediate, 6-per-deoxy-6-iodo-γ-cyclodextrin (SGMD-1);
adding methanol solution of sodium methoxide into the reaction system followed by the addition of acetone without removal of the solvents under reduced pressure to obtain the crude product of SGMD-1 as a solid after filtration;
purifying the crude SGMD-1 by recrystallization;
reacting thus obtained recrystallized intermediate (SGMD-1) with 3-mercaptopropionic acid (SM2) in basic medium e.g., sodium hydride, to obtain a crude product of 6-per-deoxy-6-per-(2-carboxyethyl)thio -γ-cyclodextrin sodium salt (SGMD);
purifying the crude SGMD by passing through adsorbents followed by recrystallization.

In an embodiment of the present invention, the organic solvent is N,N-dimethylformamide.

In an embodiment of the present invention, t process for the preparation of Sugammadex sodium is outlined as follows:

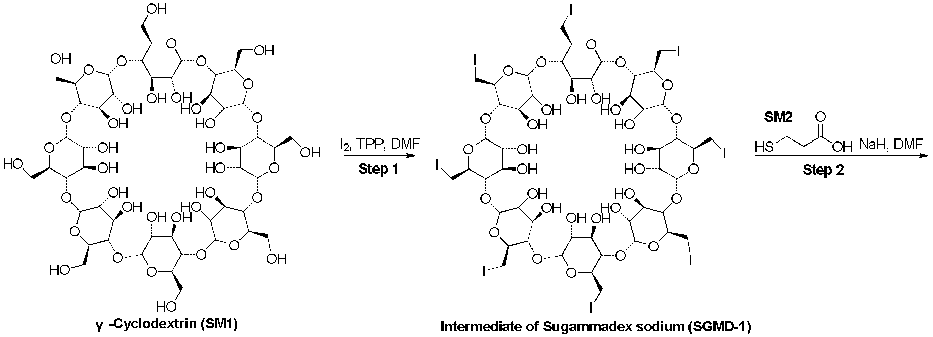

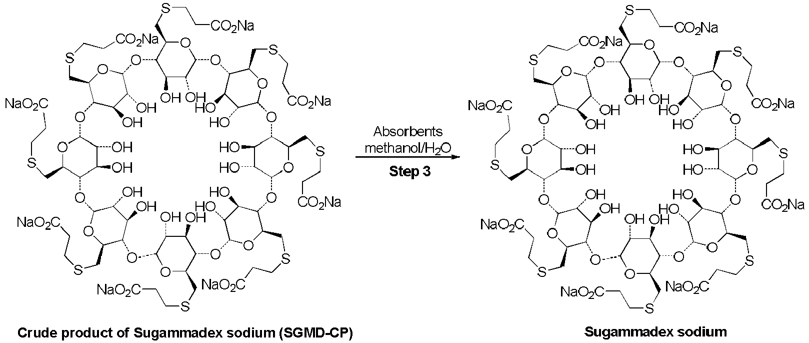

In an embodiment of the present invention, the ratio (V/W) of acetone and the γ- cyclodextrin (SM1) is 30:1 ∼ 150:1, preferably 35:1∼140:1 , 40:1∼130:1 , 45:1∼120:1, 50:1∼110:1 , 50:1∼100:1, and most preferably 60:1∼100:1.

In an embodiment of the present invention, the process is characterized in that prior to the preparation of crude SGMD, the obtained intermediate SGMD-1 is precipitated by the addition of acetone to the reaction system which is then purified by recrystallization. Solvent used in the recrystallization of SGMD-1 is dimethylformamide (DMF), dimethyl sulfoxide (DMSO), methanol, ethanol, isopropanol or acetone, or the mixture of the two above solvents, preferably a mixture of acetone and DMF, a mixture of acetone and DMSO, a mixture of methanol and DMF or a mixture of ethanol and DMF, and most preferably a mixture of acetone and DMF. Theratio (V/V) of the mixture of the two above solvents is 1:0.3∼1:2.5, preferably 1:0.4∼1:2.4, 1:0.5∼1:2.3, 1:0.6∼1:2.2, 1:0.7∼1:2.1, and most preferably 1:0.8∼1:2.0.

In an embodiment of the present invention, the process is characterized in that the molar ratio of intermediate 1 (SGMD-1) and 3-mercaptopropionic acid (SM2) is 1:8∼1:25, preferably 1:9∼1:24, 1:10∼1:22, 1:11∼1:21, and most preferably 1:12∼1:20.

In an embodiment of the present invention, the process is characterized in that the molar ratio of intermediate 1 (SGMD-1) and sodium hydride is 1:10 ∼ 1:50, preferably 1:12∼1:48, 1:15∼1:45, 1:17∼1:42, 1:18∼1:40, and most preferably 1:22∼1:40.

In an embodiment of the present invention, the process is characterized in that the solvent used in the recrystallization of crude SGMD is ethanol, water, methanol or isopropanol, or a mixture of the two above solvents, preferably a mixture of methanol and water or a mixture of ethanol and water.

In an embodiment of the present invention, the process is characterized in that the adsorbent is active carbon, silica gel, macroporous resin, aluminum oxide (basic aluminum oxide or neutral aluminum oxide), molecular sieves or zeolite, or the combination of 2∼3 above adsorbents, preferably the combination of aluminum oxide and active carbon, wherein aluminum oxide and active carbon could be used alone or in combination.

In an embodiment of the present invention, the characteristic of the method is that the ratio (W/W) of crude SGMD and absorbent(s) is 1:0.1 ∼ 1:2.5, preferably 1:0.1∼1:2.3, 1:0.1∼1:2.1, 1:0.2∼1:2.0 or 1:0.2∼1:1.8, and most preferably 1:0.2∼1:1.5.

Compared to the prior art, the present invention avoids the influence of triphenylphosphine oxide, a byproduct caused by the reaction between γ-cyclodextrin and iodine in the presence of triphenylphosphineon the following reactions, evaporation of high boiling point solvent DMF so that to simplify the process and improve the quality of SGMD-1. Meanwhile, the process of the present invention is simple and economic by removing impurities from crude SGMD with absorbent(s) and recrystallizing crude SGMD, which can also provide desired Sugammadex sodium salt without new impurities. Therefore, the quality of Sugammadex sodium salt produced by the present process is controllable which is comparable to the quality of the commercially available product.

### Descriptionof Drawings

Figure 1: ¹H-NMR of 6-per-deoxy-6-per-iodo -γ-cyclodextrin.
Figure 2: HRMS of 6-per-deoxy-6-per-iodo -γ-cyclodextrin.
Figure 3: ¹H-NMR of 6-per-deoxy-6-per-(2-carboxyethyl)thio-γ-cyclodextrin Sodium Salt.
Figure 4: HRMS of 6-per-deoxy-6-per-(2-carboxyethyl)thio-γ-cyclodextrin Sodium Salt.
Figure 5: Analysis spectrum of the related substances of sugammadex sodium prepared by the process of the present invention.
Figure 6: Analysis spectrum of the related substances of commercially available Injection Bridion 5ml (batch number;R501G).
Figure 7: Analysis spectrum of the related substances of commercially available Injection Bridion 5ml (batch number;S217P).
Figure 8: Analysis spectrum of the related substances of commercially available Injection Bridion 5ml (batch number;S502P) .

### Embodiments

Detailed embodiments of the present invention are disclosed herein below. However, it is to be understood that the disclosed embodiments are merely exemplary of the invention. The scope of the invention is not limited to the disclosed embodiments.

### EXAMPLE 1: Preparation of 6-per-deoxy-6-per-iodo-γ-cyclodextrin (SGMD-1)

To a 1L three-necked flask, dimethyformamide (DMF) (170 g) and triphenylphosphine (36.16 g) were introduced sequentially with stirring under an atmosphere of nitrogen at room temperature. The mixture was stirred till the triphenylphosphine was completely dissolved. To the above mixture was added dropwise a solution of iodine in DMF (36.63 g of iodine in 45 g of DMF). The reaction system was maintained and stirred at 20∼30 °C for 30 min prior to the addition of γ-cyclodextrin (12 g). Then the reaction system was heated to 70 °C and stirred at the same temperature till the starting material was completely consumed (∼ 24 hrs, monitored by HPLC).

The reaction system was cooled down to 20 °C and maintained at 20∼30°C, to which a solution of sodium methoxide in methanol (8.74 g of sodium methoxide suspended in 48 g of methanol) was added dropwise. The mixture was stirred for 2 hrs at the same temperature prior to the addition of acetone (995 g) during the course of which solid started to precipitate. The stirring was continued for another 2 hrs. The resultant solid was collected by filtration under reduced pressure, washed with acetone (20 g) and dried at 45-50 °C for 8∼13 hrs.

The resultant solid was dissolved in a premixed solvent DMF-acetone (170 g, acetone/DMF=1:0.8, V/V) at 50 °C and stirred for 60 mins at the same temperature. The reaction system was to cooled down to 20∼30 °C with stirring. The resultant crystals were filtered, washed with acetone (32 g) and dried under vacuum at 45∼50 °C for 8∼13 hrs to afford 14.9 g of the entitled compound SGMD-1 as off-white powder. Yield: 69.5%.

### EXAMPLE 2: Preparation of 6-per-deoxy-6-per-iodo-γ-cyclodextrin (SGMD-1)

To a 5L three-necked flask, dimethylformamide (DMF) (227g) and triphenylphosphine (36.16 g) were introduced sequentially with stirring under an atmosphere of nitrogen at room temperature. The mixture was stirred till the triphenylphosphine was completely dissolved. To the above mixture was added dropwise a solution of iodine in DMF (36.63 g of iodine in 45 g of DMF). The reaction system was maintained and stirred at 20∼30 °C for 30 min prior to the addition of γ-cyclodextrin (12 g). Then the reaction system was heated to 70 °C and stirred at the same temperature till the starting material was completely consumed (∼ 24h, monitored by HPLC).

The reaction system was cooled down to 20 °C and maintained at 20∼30°C, to which sodium methoxide (8.74 g sodium methoxide suspended in 48 g methanol) was added dropwise. The mixture was stirred for 2 hrs at the same temperature prior to the addition of acetone (948 g) during the course of which solid started to precipitate. The stirring was continued for another 2 hrs at 20∼30 °C. The resultant solid was collected by filtration under reduced pressure, washed with acetone (20 g), and dried at 45∼50 °C for 8∼13 hrs.

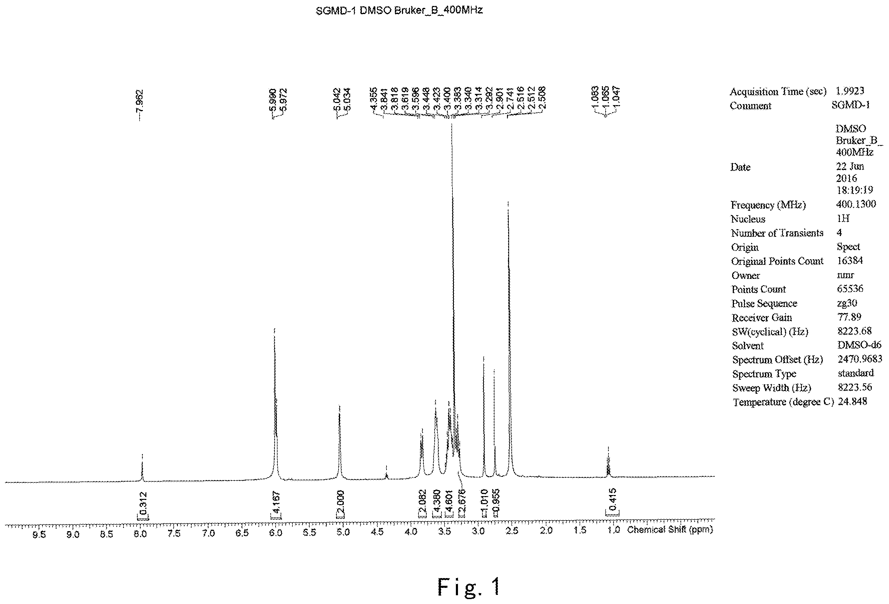

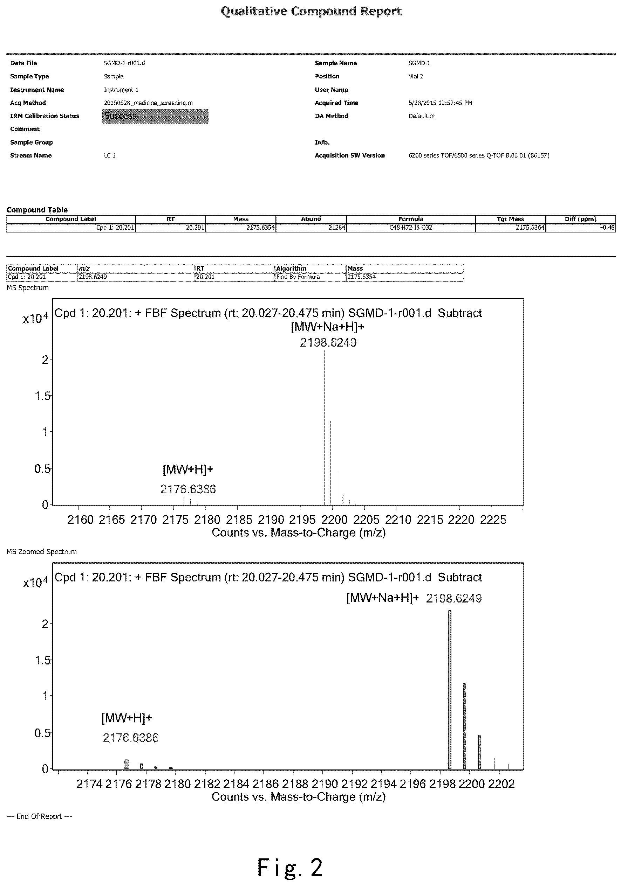

The solid was dissolved in a premixed solvent DMF-acetone (270 g, acetone/DMF=1:1.5, V/V) at 50 °C and stirred for 60 mins at the same temperature. The reaction system was cooled down to 20∼30 °C with stirring. The resultant crystals were filtered, washed with acetone(32 g) and dried under vacuum at 45∼50°C for 8∼13hrs to afford 18.72 g of the entitled compound SGMD-1 as off-white powder. Yield: 93.0%. ¹H-NMR (400 MHz, DMSO-*d*₆) δ: 5.990∼5.972 (m, 16H), 5.042∼5.034 (d, *J* = 3.2 Hz, 8H), 3.841∼3.818 (m, 8H), 3.619 (m, 16H), 3.448∼3.423 (m, 8H), 3.340∼3.292 (m, 8H). (Figure 1). ESI-HRMS: found 2176.6386 [M+H]⁺, 2198.6249 [M+Na+H]⁺, calcd: 2175.6354 [M+H]⁺, the absolute error is : 0.48 ppm, the error is acceptable. (Figure 2) .

### EXAMPLE 3: Preparation of 6-per-deoxy-6-per-iodo-γ-cyclodextrin (SGMD-1)

To a 1L three-necked flask, dimethyformamide(DMF) (159 g) and triphenylphosphine (36.16 g) were introduced sequentially with stirring under an atmosphere of nitrogen at the room temperature. The mixture was stirred till the triphenylphosphine was completely dissolved. To the above mixture was added dropwise a solution of iodine in DMF (36.63 g of iodion in 45 g of DMF). The reaction system was maintained and stirred at 20∼30 °C for 30 min prior to the addition of γ-cyclodextrin (12 g). Then the reaction system was heated to 70°C and stirred at the same temperature till the starting material was completely consumed (∼24 hrs, monitored by HPLC).

The reaction system was cooled down to 20 °C and maintained at 20∼30°C, to which a solution of sodium methoxide in methanol (8.74 g of sodium methoxide suspended in 48 g of methanol) was added. The mixture was stirred for 2 hrs at the same temperature prior to the addition of acetone (398 g) during the course of which solid started to precipitate. The stirring was continued for another 2 hrs. The resultant solid was collected by filtration under reduced pressure, washed with acetone (20 g) and dried at 45∼50 °C under vacuum for 8∼13 hrs.

The solid was dissolved in premixed solvent of DMF-acetone (270g, acetone: DMF=1.0:2.0,V/V) at 50 °C and stirred for 60 mins at the same temperature. The reaction system was cooled down to 20∼30 °C with stirring. The resultant crystals were filtered, washed with acetone (32 g) and dried under vacuum at 45∼50°C for 8∼13 hrs to afford 17.51 g of the entitled compound SGMD-1 as off-white powder. Yield: 87.0%.

### EXAMPLE 4: Preparation of 6-per-deoxy-6-per-iodo-γ-cyclodextrin (SGMD-1)

To a 50L three-necked flask, dimethyformamide(DMF) (13.2 kg) and triphenylphosphine (3.6 kg) were introduced sequentially with stirring under an atmosphere of nitrogen. The mixture was stirred till the triphenylphosphine was completely dissolved. To the above mixture was added dropwise a solution of iodine in DMF (3.67 kg iodine in 4.5 kg DMF). The reaction system was maintained and stirred at 20∼30 °C for 30 minutes prior to the addition of γ-cyclodextrin (1.2 kg). Then the reaction system was heated to 70 °C and stirred at the same temperature till the starting material was completely consumed (∼ 24 hrs, monitored by HPLC).

The reaction system was cooled down to 20 °C and maintained at 20∼30°C, to which a solution of sodium methoxide in methanol (8.74 kg of sodium methoxide suspended in 4.80 kg of methanol) was added dropwise. The mixture was stirred for 2 hrs at the same temperature prior to the addition of acetone (49.9 kg) during the course of which solid started to precipitate. The stirring was continued for another 2 hrs. The resultant solid was collected by filtration under reduced pressure, washed with acetone (2.0 kg) and dried at 45-50 °C for 8∼13 hrs.

The solid was dissolved in a premixed solvent of DMF- acetone (2.6 kg, acetone:DMF=1.0:0.8, V/V) at 50 °C and stirred for 60 mins at the same temperature. The reaction system was cooled down to 20∼30 °C with stirring. The resultant crystals were filtered, washed with acetone (3.2 kg) and dried at 45-50 °C under vacuum for 8∼13 hrs to afford 1.77 kg of the entitled compound SGMD-1 as off-white powder. Yield: 87.9%.

### EXAMPLE 5: Preparation of 6-per-deoxy-6-per-(2-carboxyethyl) thio-γ-cyclodextrin Sodium Salt (SGMD).

To a 1L three-necked flask, DMF (200 g) and 3-mercaptopropionic acid (SM2, 5.76 g) were added successively under an atmosphere of of nitrogen. After the reaction system was cooled down to 0-5 °C, sodium hydride (7.38 g) was added. The reaction system was maintained and vigorously stirred for 30 mins. Then a solution of SGMD-1 in DMF (10 g of SGMD-1 dissolved in 66.2 g of DMF) was added dropwise over a period of 20∼40 mins. Then the reaction system was heated to 70∼75 °C and stirred at the same temperature till the SGMD-1 was completely consumed (∼12 hrs).

The resultant mixture was cooled down to 20 -30 °C, to which purified water (96 g) was added and stirred for a further 30 mins at the same temperature. The precipitated solid was filtered, washed with acetone (20 g) and dried at 45±2°C for 12∼15 hrs to afford 8.81 g of the crude product SGMD. Yield: 88.1%.

### EXAMPLE 6: Preparation of 6-per-deoxy-6-per-(2-carboxyethyl)thio-γ-cyclodextrin Sodium Salt

To a 1L three-necked flask, DMF (200 g) and 3-mercaptopropionic acid (SM2, 9.64 g) were added successively under an atmosphere of nitrogen. After the reaction system was cooled down to 0-5 °C, sodium hydride (7.38 g) was added. The reaction system was maintained and vigorously stirred for 30 mins. To above reaction mixture was then added dropwise a solution of SGMD-1 in DMF (SGMD-1 (10g) dissolved in DMF (66.2 g)) over a period of 20-40 min. Then the reaction system was heated to 70∼75 °C and stirred at the same temperature till the SGMD-1 was completely consumed (∼12 hrs).

The reaction mixture was cooled down to 20∼30 °C, to which purified water (96 g) was added and stirred for a further 30 mins. The precipitated solid was filtered, washed with acetone (20 g) and dried at 45±2 °C for 12∼15 hrs to afford 8.91 g of the crude product SGMD as solid. Yield: 84.1%.

### EXAMPLE 7: Preparation of 6-per-deoxy-6-per-(2-carboxyethyl)thio-γ-cyclodextrin Sodium Salt

To a 1L three-necked flask, DMF (200 g) and 3-mercaptopropionic acid (SM2, 8.64 g) were added successively under an atmosphere of nitrogen. The reaction system was cooled down to 0∼5 °C, to which sodium hydride (6.42 g) was added and vigorously stirred for 30 mins. To above reaction mixture was added dropwise a solution of SGMD-1 in DMF (SGMD-1(10 g) dissolved in DMF (66.2 g)) over 20∼40 mins. Then the reaction system was heated to 70-75 °C and stirred at the same temperature till the SGMD-1 was completely consumed (∼12 hrs).

The reaction mixture was cooled down to 20∼30 °C, to which purified water (96 g) was added and stirred for a further 30 mins. The precipitated solid was filtered, washed with acetone (20 g) and dried at 45±2 °C for 12∼15 hrs to afford 9.02 g of the crude product SGMD as solid. Yield: 90.2%.

### EXAMPLE 8: Preparation of 6-per-deoxy-6-per-(2-carboxyethyl)thio-γ-cyclodextrin Sodium Salt

To a 1L three-necked flask, DMF (200 g) and 3-mercaptopropionic acid (SM2, 8.64 g) were added successively under an atmosphere of nitrogen. The reaction system was cooled down to 0∼5 °C, to which sodium hydride (4.41 g) was added with vigorous stirring for 30 mins. To above reaction mixture was added dropwise a solution of SGMD-1 in DMF (SGMD-1(10 g) dissolved in DMF (66.2 g)) over 20∼40 mins. Then the reaction system was heated to 70-75 °C and stirred at the same temperature till the SGMD-1 was completely consumed (∼12 hrs).

The reaction mixture was cooled down to 20∼30 °C, to which purified water (96 g) was added and stirred for a further 30 mins. The precipitated solid was filtered, washed with acetone (20 g) and dried at 45±2 °C for 12∼15 hrs to afford 9.34 g of the crude product SGMD as solid. Yield: 93.4%.

### EXAMPLE 9: Preparation of 6-per-deoxy-6-per-(2-carboxyethyl)thio-γ-cyclodextrin Sodium Salt

To a 50L reaction kettle, DMF (15.88 kg) and 3-mercaptopropionic acid (SM2, 1.05 kg) were added successively under an atmosphere of nitrogen. The reaction system was cooled down to 0∼5 °C, to which sodium hydride (7.38 g) was added with vigorous stirring for 30 mins. To above reaction mixture was added dropwise a solution of SGMD-1 in DMF (SGMD-1 (1.2 kg) dissolved in DMF (7.94 kg)) over 20∼40 mins. Then the reaction system was heated to 70-75 °C and stirred at the same temperature till the SGMD-1 was completely consumed (∼12 hrs).

The reaction mixture was cooled down to 20∼30 °C, to which purified water (3.84 kg) was added and stirred for a further 30 mins. The precipitated solid was filtered, washed with acetone (7.8 kg) and dried at 45±2 °C for 12∼15 hrs to afford 1.1 kg of the crude product SGMD as solid. Yield: 91.7%.

### EXAMPLE 10: Preparation of 6-per-deoxy-6-per-(2-carboxyethyl)thio-γ-cyclodextrin Sodium Salt

To a 1L three-necked flask, DMF (200 g) and 3-mercaptopropionic acid (SM2, 5.76 g) were added successively under an atmosphere of nitrogen. The reaction system was cooled down to 0∼5 °C, to which sodium hydride (4.06 g) was added with vigorous stirring for 30 mins. To above reaction mixture was added dropwise a solution of SGMD-1 in DMF (SGMD-1(10 g) dissolved in DMF (66.2 g)) over 20∼40 mins. Then the reaction system was heated to 70-75 °C and stirred at the same temperature till the SGMD-1 was completely consumed (∼12 hrs).

The reaction mixture was cooled down to 20∼30 °C, to which purified water (96 g) was added and stirred for a further 30 mins. The precipitated solid was filtered, washed with acetone (20 g) and dried at 45±2 °C for 12∼15 hrs to afford 8.91 g of the crude product SGMD as solid. Yield: 89.1%.

### EXAMPLE 11: Purification of 6-per-deoxy-6-per-(2-carboxyethyl)thio-γ-cyclodextrin Sodium Salt (SGMD).

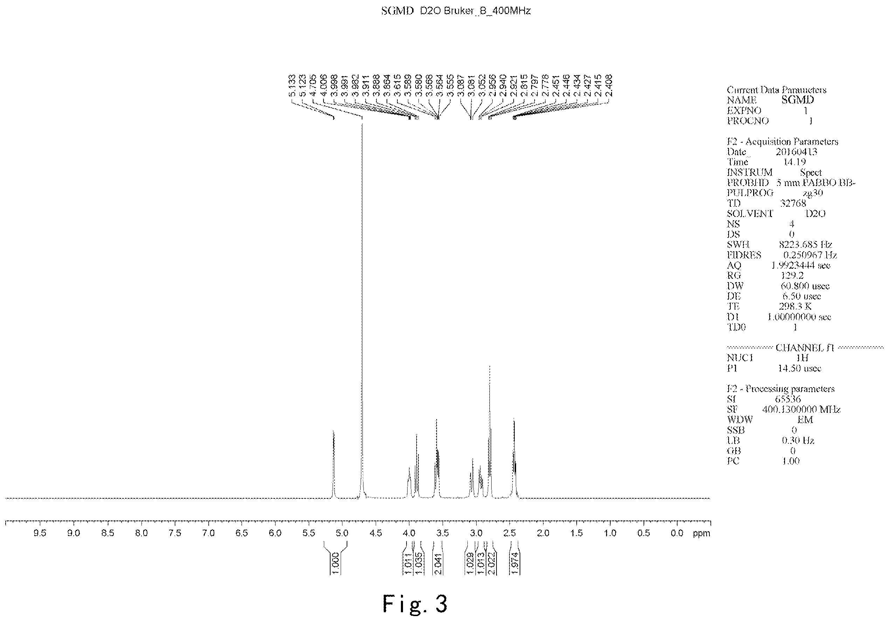

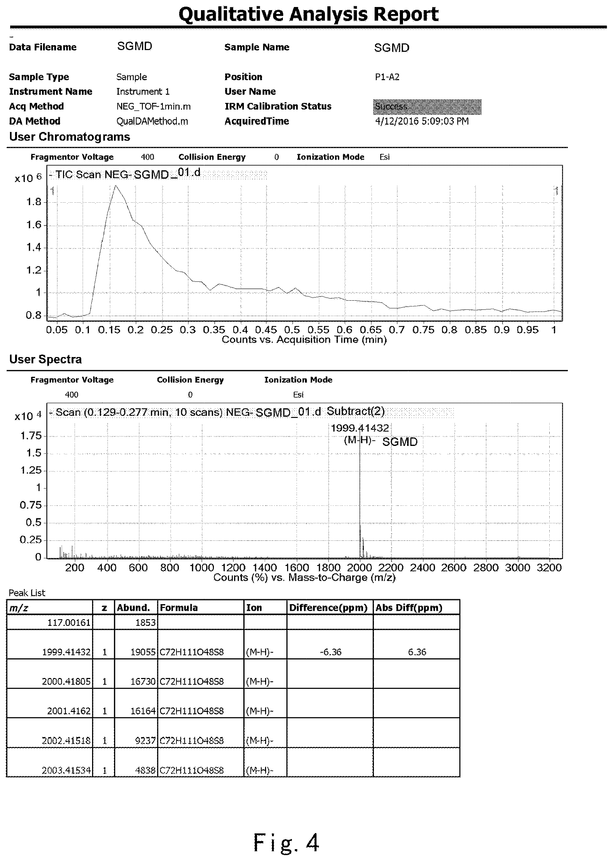

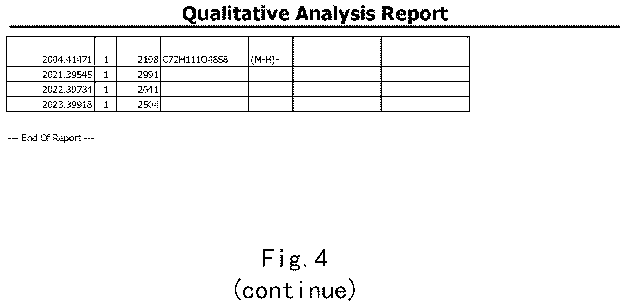

The crude product of SGMD (50 g) was dissolved in a premixed solvent of water(150 g)-methanol (100 g) and heated to 50 °C. At this temperature, active carbon (75 g) was added and stirred for 30 mins. Then the active carbon was filtered and washed with purified water (50 g). The resultant filtrate was heated to 50-55 °C under the atmosphere of nitrogen, to which methanol (200 g) was added dropwise. After the addition, the reaction system was slowly cooled down to 25∼30 °C and stirred at the same temperature for a further 30 mins. The solid was filtered, washed with methanol(100 g) and dried at 60∼65 °C for 24 hrs to afford 30.5 g of the pure product (SGMD) as white powder. Yield: 61.0%. ¹H-NMR (400 MHz, D₂O) δ: 5.123∼5.133 (d, *J* = 4 Hz, 8H), 3.982∼4.006 (m, 8H), 3.864∼3.911 (m, 8H), 3.555∼3.615 (m, 16H), 3.052∼3.087 (m, 8H), 2.921∼2.956 (m, 8H), 2.778∼2.815 (m, 16H), 2.408∼2.451 (m, 16H) (Figure 3) ; ESI-HRMS: calcd for [M-H]⁻ 1999.40159, found 1999.41432, the absolute error is 6.36 ppm and acceptable(Figure 4). Impurity profile of Sugammadex sodium prepared in this example are shown in line SG11, Table 1. The purity of principal peaks is 98.842% (quantitatively by area normalization method). All related substances are acceptable based on the Bridion's acceptance criteria (Shown in Table 1).

### EXAMPLE 12: Purification of 6-per-deoxy-6-per-(2-carboxyethyl)thio-γ-cyclodextrin Sodium Salt

The crude SGMD (50 g) was dissolved in a premixed solvent of water (150 g) - ethanol (150 g) and heated to 50 °C. Active carbon (10 g) and Al₂O₃ (50 g) were added and stirred for 30 mins at this temperature. Then the mixture was filtered and the solid cake was washed with purified water (50 g). The resultant filtrate was heated to 50-55 °C under the atmosphere of nitrogen, to which ethanol (200 g) was added dropwise. After the addition, the mixture was slowly cooled to 25∼30 °C and stirred for a further 30 mins at the same temperature. The mixture was filtered, washed the solid cake with ethanol (150 g) and dried at 60∼65 °C for 24 hrs to afford 27.8 g of the pure product as white powder. Yield: 55.6%. Impurity profile of Sugammadex sodium prepared in this example are shown in line SG12, Table 1. The purity of principal peaks is 98.488% (quantitatively by area normalization method). All related substances are acceptable based on the Bridion's acceptance criteria (Shown in Table 1).

### EXAMPLE 13: Purification of 6-per-deoxy-6-per-(2-carboxyethyl)thio-γ-cyclodextrin Sodium Salt

The crude SGMD (50 g) was dissolved in premixed solution of purified water (150 g)- ethanol (150 g) and heated to 50 °C, to which Al₂O₃ (75 g) was added and stirred for 30 mins. Then the mixture was filtered and the solid cake was washed with ethanol (50 g). The resultant filtrate was heated to 50-55 °C under the atmosphere of nitrogen, to which ethanol (400 g) was added dropwise. After addition, the mixture was slowly cooled to 25∼30 °C and stirred for a further 30 mins at the same temperature. The precipitate solid was filtered, washed with ethanol (150 g) and dried at 60∼65 °C for 24 hrs to afford 23.6 g of entitled SGMD as white powder. Yield: 47.2%. Impurity profile of Sugammadex sodium prepared in this example are shown in line SG13, Table 1. The purity of principal peaks is 98.734% (quantitatively by area normalization method). All related substances are acceptable based on the Bridion's acceptance criteria (Shown in Table 1).

### EXAMPLE 14: Purification of 6-per-deoxy-6-per-(2-carboxyethyl)thio-γ-cyclodextrin Sodium Salt

The crude SGMD (50 g) was dissolved in premixed solution of purified water (150 g)- methanol (100 g) and heated to 50 °C, to which Al₂O₃ (60 g) was added and stirred for 30 mins. Then the mixture was filtered and the solid cake was washed with purified water (50 g). The resultant filtrate was heated to 50-55 °C under the atmosphere of nitrogen, to which methanol (200 g) was added dropwise. After addition, the mixture was slowly cooled to 25∼30 °C and stirred for a further 30 mins at the same temperature. The precipitate solid was filtered, washed with methanol (100 g) and dried at 60∼65 °C for 24 hrs to afford 35.1 g of entitled SGMD as white powder. Yield: 70.2%. Impurity profile of Sugammadex sodium prepared in this example are shown in line SG14, Table 1. The purity of principal peaks is 98.790% (quantitatively by area normalization method). All related substances are acceptable based on the Bridion's acceptance criteria (Shown in Table 1).

### EXAMPLE 15: Purification of 6-per-deoxy-6-per-(2-carboxyethyl)thio-γ-cyclodextrin Sodium Salt

The crude SGMD (50 g) was dissolved in premixed solution of purified water (150 g)-methanol (150 g) and heated to 50 °C, to which active carbon (40 g) was added and stirred for 30 mins. Then the mixture was filtered and the solid cake was washed with purified water (50 g). The resultant filtrate was heated to 50-55 °C under the atmosphere of nitrogen, to which methanol (200 g) was added dropwise. After addition, the mixture was slowly cooled to 25∼30 °C and stirred for a further 30 mins at the same temperature. The precipitate solid was filtered, washed with methanol (100 g) and dried at 60∼65 °C for 24 hrs to afford 31.6 g of entitled SGMD as white powder. Yield: 63.2%. Impurity profile of Sugammadex sodium prepared in this example are shown in line SG15, Table 1. The purity of principal peaks is 98.884% (quantitatively by area normalization method). All related substances are acceptable based on the Bridion's acceptance criteria (Shown in Table 1).

### EXAMPLE 16: Purification of 6-per-deoxy-6-per-(2-carboxyethyl)thio-γ-cyclodextrin Sodium Salt

The crude SGMD (50 g) was dissolved in premixed solution of purified water (150 g)- methanol (100 g) and heated to 50 °C, to which active carbon (10 g) and neutral Al₂O₃ (65 g) were added and stirred for 30 mins. Then the mixture was filtered and the solid cake was washed with purified water (50 g). The resultant filtrate was heated to 50-55 °C under the atmosphere of nitrogen, to which methanol (200 g) was added dropwise. After addition, the mixture was slowly cooled to 25-30 °C and stirred for a further 30 mins at the same temperature. The precipitate solid was filtered, washed with methanol (100 g) and dried at 60∼65 °C for 24 hrs to afford 34.5 g of entitled SGMD as white powder. Yield: 69.0%. Impurity profile of Sugammadex sodium prepared in this example are shown in line SG16, Table 1. The purity of principal peaks is 98.833% (quantitatively by area normalization method). All related substances are acceptable based on the Bridion's acceptance criteria (Shown in Table 1).

### EXAMPLE 17: Purification of 6-per-deoxy-6-per-(2-carboxyethyl)thio-γ-cyclodextrin Sodium Salt

The crude SGMD (50 g) was dissolved in a premix of water (150 g)-methanol (100 g) and heated to 50 °C, to which active carbon (10 g) was added and stirred for 30 mins. Then the active carbon was filtered and washed with purified water (50 g). The resultant filtrate was heated to 50-55 °C under the atmosphere of nitrogen, to which methanol (200 g) was added dropwise. After addition, the mixture was slowly cooled to 25∼30 °C and stirred for a further 30 mins at the same temperature. The precipitate was filtered, washed with methanol (100 g) and dried at 60∼65 °C for 24 hrs to afford 36.2 g of entitled product as white powder. Yield: 72.4%. Impurity profile of Sugammadex sodium prepared in this example are shown in line SG17, Table 1. The purity of principal peaks is 98.878% (quantitatively by area normalization method). All related substances are acceptable based on the Bridion's acceptance criteria (Shown in Table 1).

### EXAMPLE 18: Purification of 6-per-deoxy-6-per-(2-carboxyethyl)thio-γ-cyclodextrin Sodium Salt

The crude SGMD 50g was dissolved in a premix solution of water (150 g)-methanol (100 g) and heated to 50 °C, to which active carbon (20 g) and neutral Al₂O₃ (40 g) were added and stirred for 30 mins. Then the mixture was filtered and the solid cake was washed with purified water (50 g). The resultant filtrate was heated to 50-55 °C under the atmosphere of nitrogen, to which methanol (200 g) was added dropwise. After addition, the mixture was slowly cooled to 25∼30 °C and stirred for a further 30 mins. The mixture was filtered, washed with methanol (100 g) and dried at 60∼65 °C for 24 hrs to afford 33.4 g of title product as white powder. Yield: 66.8%. Impurity profile of Sugammadex sodium prepared in this example are shown in line SG18, Table 1. The purity of principal peaks is 98.783% (quantitatively by area normalization method). All related substances are acceptable based on the Bridion's acceptance criteria (Shown in Table 1).

### EXAMPLE 19: Purification of 6-per-deoxy-6-per-(2-carboxyethyl)thio-γ-cyclodextrin Sodium Salt

The crude SGMD (50g) was dissolved in a premix solution (water (150 g)- ethanol(150 g)) and heated to 50°C, to which neutral Al₂O₃ (25 g) was added and stirred for 30 mins. Then the mixture was filtered and the solid cake was washed with purified water (50 g). The resultant filtrate was heated to 50-55 °C under the atmosphere of nitrogen, to which ethanol (200 g) was added dropwise. After addition, the mixture was slowly cooled to 25∼30 °C and stirred for a further 30 mins. The mixture was filtered, washed with ethanol (150 g) and dried at 60∼65 °C for 24 hrs to afford 43.3 g of title product as white powder. Yield: 86.6%. Impurity profile of Sugammadex sodium prepared in this example are shown in line SG19, Table 1. The purity of principal peaks is 99.269% (quantitatively by area normalization method). All related substances are acceptable based on the Bridion's acceptance criteria (Shown in Table 1).

### EXAMPLE 20: Purification of 6-per-deoxy-6-per-(2-carboxyethyl)thio-γ-cyclodextrin Sodium Salt

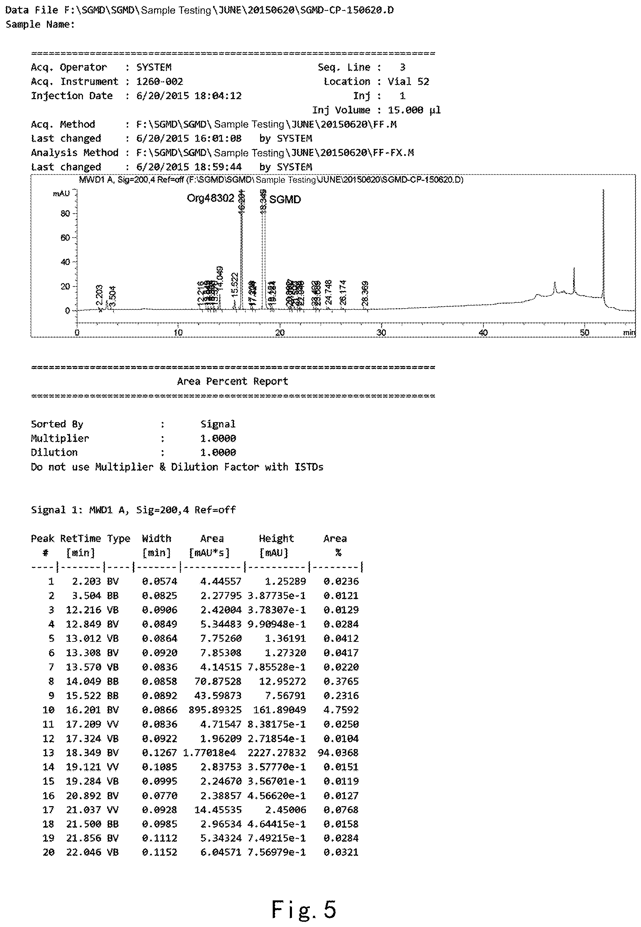

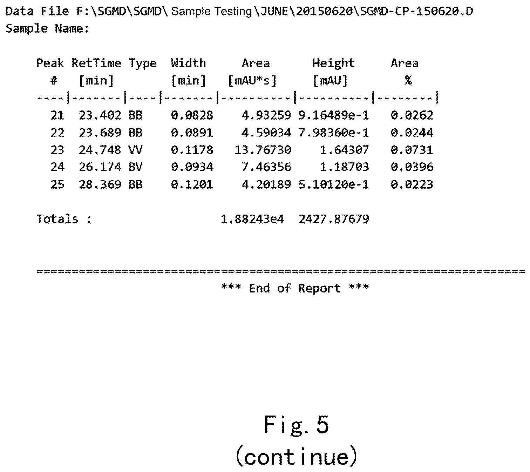

The crude SGMD (1.5 kg)was dissolved in a premix solution (water (4.5 kg)- methanol (3.0 kg)) and heated to 50 °C, to which active carbon (300 g) and neutral Al₂O₃ (750 g) were added and stirred for 30 mins. Then the mixture was filtered and the solid cake was washed with purified water (1.5 kg). The resultant filtrate was heated to 50-55 °C under the atmosphere of nitrogen, to which methanol (6.0 kg) was added dropwise. After addition, the mixture was slowly cooled to 25∼30 °C and stirred for a further 30 mins. The mixture was filtered, washed with methanol (3.0 kg) and dried at 60∼65 °C for 24 hrs to afford 0.95 kg title product as white powder. Yield: 63.3%.Impurity profile of Sugammadex sodium prepared in this example are shown in line SG20, Table 1 (Figure 5, The detection of impurities can refer to the section "Impurity Analysis of Sugammadex sodium salt prepared by this process and reference listed drug (Bridion)"). The purity of principal peaks is 98.796% (quantitatively by area normalization method). All related substances are acceptable based on the Bridion's acceptance criteria (Shown in Table 1).

### EXAMPLE 21: Purification of 6-per-deoxy-6-per-(2-carboxyethyl)thio-γ-cyclodextrin Sodium Salt

The crude SGMD (1.5 kg) was dissolved in a premix solution of water (4.5 kg) - ethanol (4.5 kg) and heated to 50 °C, to which active carbon (150 g) and neutral Al₂O₃ (2.25 kg) were added and stirred at this temperature for 30 mins. Then the mixture was filtered and the solid cake was washed with purified water (1.5 kg). The resultant filtrate was heated to 50-55 °C under the atmosphere of nitrogen, to which ethanol (9 kg) was added dropwise at the same temperature. After addition, the reaction system was slowly cooled to 25∼30 °C and stirred for a further 30 mins. The mixture was filtered, washed with ethanol (3 kg) and dried at 60∼65 °C for 24 hrs to afford 1.08 kg of entitled product as white powder. Yield: 72.0%. Impurity profile of Sugammadex sodium prepared in this example are shown in line SG21, Table 1. The purity of principal peaks is 98.916% (quantitatively by area normalization method). All related substances are acceptable based on the Bridion's acceptance criteria (Shown in Table 1).

### IMPURITY ANALYSIS OF SUGAMMADEX SODIUM SALT PREPARED BY THIS PROCESS AND REFERENCE LISTED DRUG (BRIDION).

Impurities or by-products introduced in the manufacture process of Sugammadex sodium have similar chemical structures and polarities with Sugammadex sodium (SGMD). The UV absorption of these impurities is the same or similar with SGMD. Therefore, in the early stage of process optimization, amount of impurities in BRIDION and SGMD prepared in examples 11 to 21 was calculated by area normalization method according to Chinese Pharmacopoeia 2015 in the case of reference substances of impurities were not available, respectively.

### SAMPLE PREPARATION

### PREPARTION OF TEST SAMPLES:

(1) Test solution of Sugammadex sodium: Transfer an accurately weighed quantity of Sugammadex sodium salt prepared in examples 11-21 to a 10 ml volumetric flask and dilute with purified water to a concentration of about 2.0 mg of Sugammadex sodium per mL. The prepared test solutions are mixed prior to test (abbreviated as SG11, SG12, SG13, SG14, SG15, SG16, SG17, SG18, SG19, SG20 and SG21, respectively).
(2) Test solution of Reference Listed Drug (Bridion): Transfer 1.0 ml of the Reference Listed Drug (Bridion purchased from Japan MSD Corporation, batch number: S217P, S502P and R501G, concentration: 100 mg/ml) to a 50 ml volumetric flask and dilute with purified water to a concentration of about 2.0 mg of Bridon per mL.

### SAMPLE ANALYSIS:

Agilent 1260 high performance liquid chromatograph (HPLC) system (purchased from Agilent Technologies, equipped with UV detector, column thermostat and autosampler) is used.

Based on Chinese Pharmacopoeia 2015, gradient elution is performed using HPLC system, wherein column is filled with octadecyl silane bonded silica gel, the detection wavelength is 200nm, the flow rate is 0.5 mL per minute, and the column temperature is set to 40 °C, the mobile phases are as follows:
Solution A: Buffer : acetonitrile (83:20, V/V), wherein buffer is 25mM sodium dihydrogen phosphate solution with pH of 3.0 which is adjusted with phosphoric acid,
Solution B: Acetonitrile

The gradients used in the detection are as follows:

| T(min) | 0 | 5 | 15 | 22 | 27 | 32 | 37 | 42 | 42.01 | 52 |
|---|---|---|---|---|---|---|---|---|---|---|
| B(%) | 0 | 0 | 2 | 8 | 25 | 50 | 70 | 70 | 0 | 0 |
| A(%) | 100 | 100 | 98 | 92 | 75 | 50 | 30 | 30 | 100 | 100 |

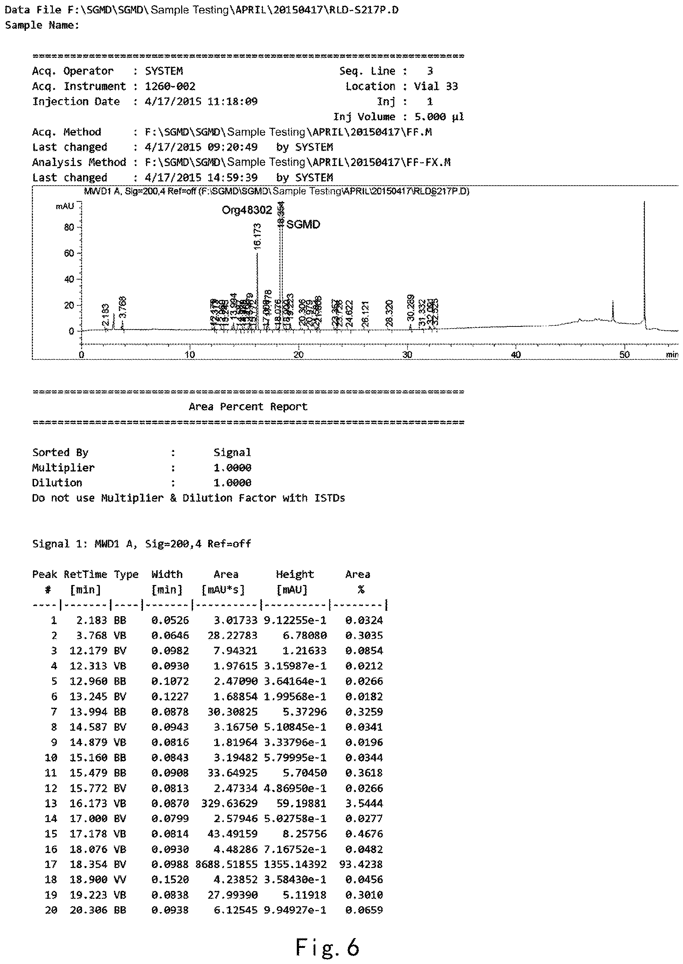

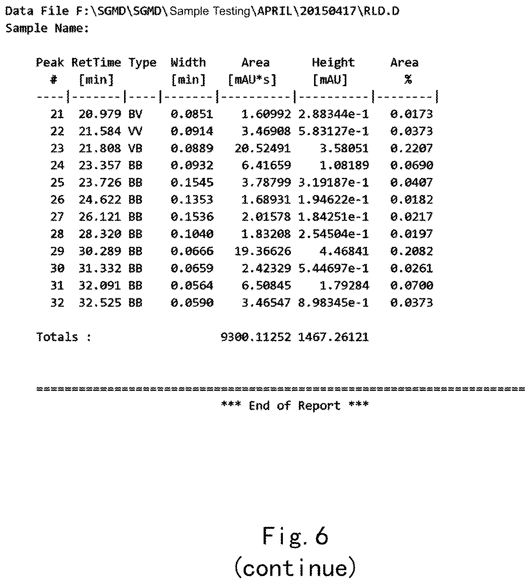

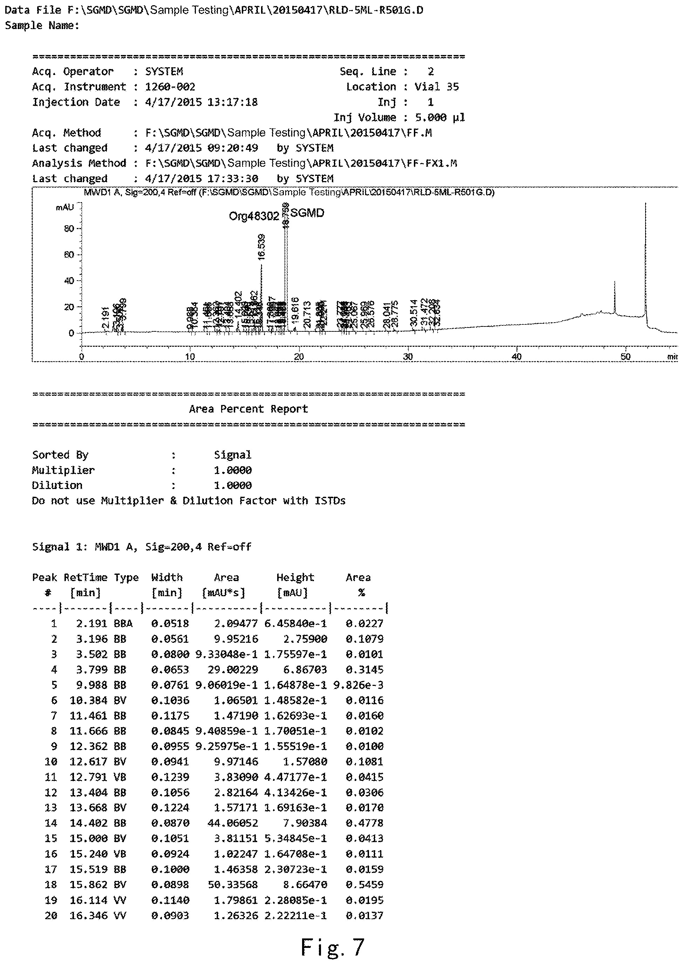

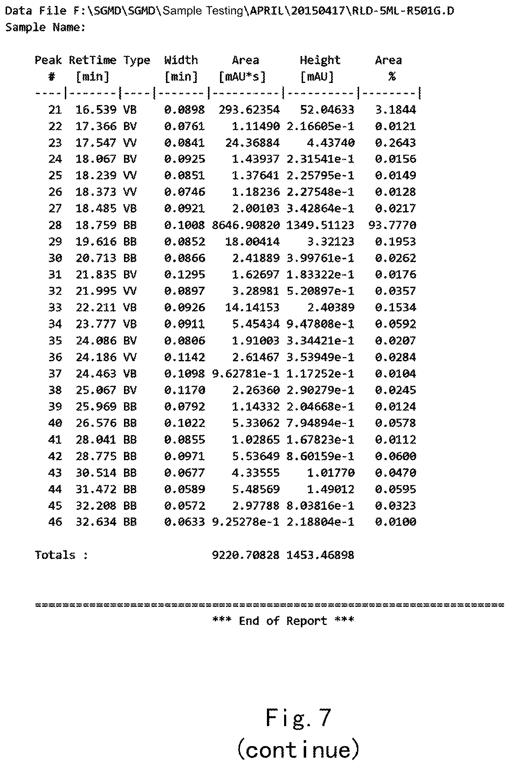

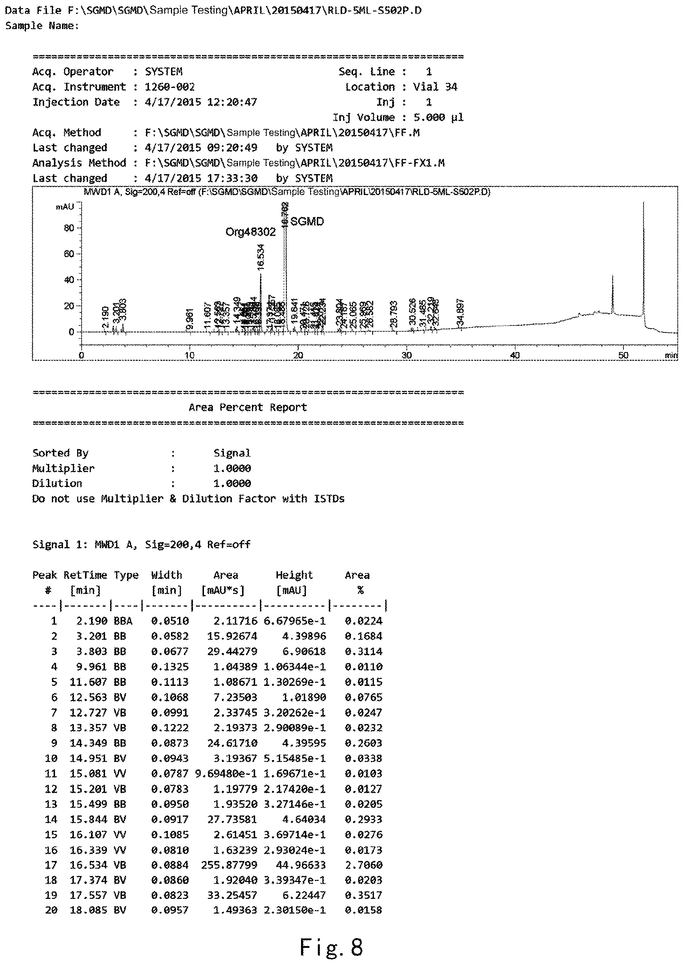

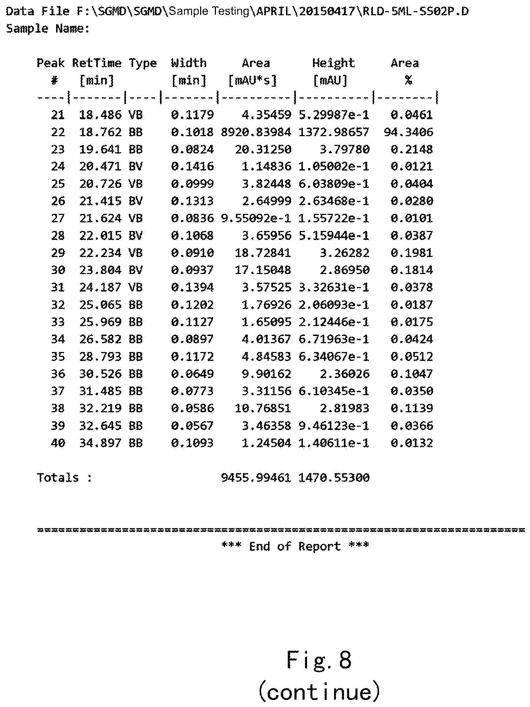

The relative retention time (RRT) is used to fix the positions of the impurities and the principal components. In the liquid chromatograph traces of the test solution of Reference Listed Drug (Bridion) and Sugammadex sodium prepared in the present invention, the peaks of which the RRTs are 0.88 and 1.00 are Org48302 and Sugammadex sodium respectively. Org48302 is regarded as a principal component too. Results of the above analysis have been summarized in Table 1. In the three batches of the Reference Listed Drug (Bridion), the purity of the two principal components by area normalized method is about 97.0%; the number of detected impurities is greater than that in the sample product prepared following the process described in this invention. The spectra of the three batches of Reference Listed Drug (Bridion) are shown in Figures 6 to 8.

It is to be illustrated that all documents mentioned in the present invention are incorporated by reference as if each individual document is individually incorporated by reference. In addition, it is to be understood that the invention has been described with reference to specific embodiments thereof and the principles of the invention, and that various changes and modifications may be effected therein by one skilled in the art without departing from the scope of the invention spirit, and scope of the invention, which equivalents fall within the scope of the invention.

**Table 1 Comparison of the Impurities of Sugammadex Sodium Prepared by Different Process**

| Name of Sample 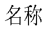 | **The normalized content of Impurities and Principal Component (%)** | | | | | | | | | | | | | | | | |
|---|---|---|---|---|---|---|---|---|---|---|---|---|---|---|---|---|---|
| | **0.17** | **0.21** | **0.66** | **0.76** | **0.84** | **0.88** | **0.94** | **0.98** | **1.00** | **1.03** | **1.05** | **1.11** | **1.19** | **1.72** | **1.53** | **1.65** | **1.75** |
| S217P | - | 0.304 | 0.085 | 0.326 | 0.362 | **3.544** | 0.468 | 0.048 | **93.424** | 0.046 | 0.301 | 0.066 | 0.221 | 0.069 | -- | 0.208 | 0.070 |
| R501G | 0.108 | 0.315 | 0.010 | 0.478 | 0.546 | **3.184** | 0.264 | 0.022 | **93**.777 | 0.195 | 0.030 | 0.153 | 0.059 | 0.058 | -- | 0.047 | 0.060 |
| S502P | 0.168 | 0.311 | 0.077 | 0260 | 0.293 | **2.706** | 0.351 | 0.046 | **94.341** | 0.215 | 0.039 | 0.198 | -- | 0.181 | 0.551 | 0.105 | 0.114 |
| SG 11 | -- | -- | -- | 0.367 | 0.238 | **4.365** | -- | 0.012 | **94.4**77 | 0.028 | -- | 0.078 | 0.047 | -- | -- | -- | -- |
| SG 12 | -- | -- | 0.024 | 0.377 | 0.506 | **3.608** | -- | 0.047 | **94.840** | 0.022 | 0.029 | -- | 0.063 | 0.046 | 0.068 | 0.065 | -- |
| SG 13 | -- | -- | 0.014 | 0.369 | 0.239 | **4.389** | -- | 0.035 | **94.345** | 0.018 | 0.010 | -- | 0.080 | -- | 0.048 | 0.024 | 0.064 |
| SG 14 | -- | -- | -- | 0.367 | 0.237 | **4.389** | -- | 0.032 | **94.401** | 0.012 | 0.013 | 0.080 | 0.046 | 0.047 | -- | -- | -- |
| SG 15 | -- | -- | 0.012 | 0.369 | 0.240 | **4.321** | -- | 0026 | **94.563** | 0022 | 0.015 | 0.082 | 0.049 | 0.054 | -- | -- | -- |
| SG 16 | -- | -- | -- | 0.367 | 0.245 | **4.323** | -- | 0.025 | **94.510** | 0.018 | 0.033 | 0.084 | 0.053 | 0.049 | -- | -- | -- |
| SG 17 | -- | -- | -- | 0.366 | 0.243 | **4.312** | -- | 0.020 | **94.566** | 0.024 | 0.014 | 0.080 | 0.053 | 0.047 | -- | -- | -- |
| SG 18 | -- | -- | -- | 0.368 | 0.239 | **4.394** | -- | 0.027 | **94.389** | 0.019 | 0.013 | 0.079 | 0.047 | 0.055 | -- | -- | -- |
| SG 19 | -- | -- | 0.014 | 0.356 | 0.234 | **4.395** | -- | 0.031 | **94.874** | 0.036 | 0.012 | 0.087 | 0.070 | 0.054 | -- | -- | -- |
| SG 20 | -- | 0.012 | 0.013 | 0.377 | 0.232 | **4.759** | -- | -- | **94.037** | 0.015 | 0.019 | 0.077 | 0.032 | 0.073 | -- | -- | -- |
| SG 21 | -- | -- | -- | 0.353 | 0.210 | 4.473 | -- | -- | 94.443 | 0.017 | 0.020 | 0.068 | 0.021 | 0.086 | 0.055 | -- | -- |

| | | | | | | | | | | | | | | | | | |
|---|---|---|---|---|---|---|---|---|---|---|---|---|---|---|---|---|---|
| **Note: Impurities with content less than 0.05% are ignored.** | | | | | | | | | | | | | | | | | |

## Claims

1. A process for the preparation of 6-per-deoxy-6-per-(2-carboxyethyl)thio-γ-cyclodextrin sodium salt, which comprises the steps of:
reacting γ-cyclodextrin (SM1) with iodine in the presence of triphenylphosphine in an organic solvent to afford an intermediate, 6-per-deoxy-6-iodo-γ-cyclodextrin (SGMD-1);
adding methanol solution of sodium methoxide into the reaction system followed by the addition of acetone without removal of the solvents under reduced pressure to obtain the crude product of SGMD-1 as a solid after filtration;
purifying the crude SGMD-1 by recrystallization;
reacting thus obtained recrystallized intermediate (SGMD-1) with 3-mercaptopropionic acid (SM2) in basic medium e.g., sodium hydride, to obtain a crude product of 6-per-deoxy-6-per-(2-carboxyethyl)thio -γ-cyclodextrin sodium salt (SGMD);
purifying the crude SGMD by passing through adsorbents followed by recrystallization.

2. The process according to claim 1, **characterized in that** the organic solvent is N,N-dimethylformamide (DMF).

3. The process according to claim 1 or 2, **characterized in that** the ratio (V/W) of acetone and γ- cyclodextrin (SM1) is 30:1 ∼ 150:1, preferably 35:1∼140:1, 40:1∼130:1, 45:1∼120:1, 50:1∼110:1, 50:1∼100:1, and most preferably 60:1 ∼100:1.

4. The process according to anyone of claim 1 or 2, **characterized in that** the solvents used in the recrystallization of crude SGMD-1 are N,N-dimethylformamide, dimethyl sulfoxide (DMSO), methanol, ethanol, isopropanol or acetone or the mixture of the two above solvents, preferably a mixture of acetone and DMF, a mixture of acetone and DMSO, or a mixture of methanol and DMF or a mixture of ethanol and DMF, and most preferably a mixture of acetone and DMF.

5. The process according to claim 4, **characterized in that** the ratio (V/V) of acetone/DMF is 1:0.3∼1:2.5, preferably 1:0.4∼1:2.4 , 1:0.5∼1:2.3 , 1:0.6∼1:2.2, 1:0.7∼1:2.1, and most preferably 1:0.8∼1:2.0.

6. The process according to anyone of claims 1 to 5, **characterized in that** the molar ratio of SGMD-1 and 3-mercaptopropionic acid (SM2) is 1:8∼1:25, preferably 1:9∼1:24, 1:10∼1:22, 1:11∼1:21, and most preferably 1:12∼1:20.

7. The process according to anyone of claims 1 to 6, **characterized in that** the molar ratio of SGMD-1 and sodium hydride is 1:10∼1:50, preferably 1:12-1:48 , 1:15-1:45 , 1:17∼1:42, 1:18∼1:40, and most preferably 1:22∼1:40.

8. The process according to anyone of claims 1 to 7, **characterized in that** the solvents used in the recrystallization of crude SGMD are ethanol, water, methanol or isopropanol or a mixture of water and one selected from the group consisting of ethanol, methanol, or isopropanol, preferably a mixture of methanol and water or a mixture of ethanol and water.

9. The process according to anyone of claims 1 to 8, **characterized in that** the adsorbents are selected from the group consisting of active carbon, silica gel, macroporous resin, aluminum oxide, molecular sieves and zeolite, preferably aluminum oxide or activated carbon or a mixture thereof, preferably the aluminum oxide is basic aluminum oxide or neutral aluminum oxide.

10. The process according to anyone of claims 1 to 9, **characterized in that** the ratio (W/W) of crude SGMD and absorbent(s) is 1:0.1 ∼ 1:2.5, preferably 1:0.1∼1:2.3, 1:0.1∼1:2.1, 1:0.2∼1:2.0, or 1:0.2∼1:1.8, and most preferably 1:0.2∼1:1.5.

## Patentansprüche

1. Verfahren zur Erzeugung von 6-Per-deoxy-6-per-(2-carboxyethyl)thio-γ-cyclodextrin-Natriumsalz, enthaltend die folgenden Schritte:
Reaktion von γ-Cyclodextrin (SM1) mit Jod in der Gegenwart von Triphenylphosphin in einem organischen Lösungsmittel, unter Erhalt eines Zwischenproduktes 6-Per-deoxy-6-jod-γ-cyclodextrin (SGMD-1),
Zugabe einer Methanollösung aus Natriummethoxid zu dem Reaktionssystem, mit anschließender Addition von Aceton ohne Entfernung der Lösungsmittel unter vermindertem Druck, unter Erhalt des rohen Produktes von SGMD-1 als Feststoff nach Filtration,
Reinigen des rohen SGMD-1 durch Rekristallisierung,
Reaktion des somit erhalten rekristallisierten Zwischenproduktes (SGMD-1) mit 3-Mercaptopropionsäure (SM2) in einem basischen Medium, z.B. Natriumhydrid, unter Erhalt eines rohen Produktes von 6-Per-deoxy-6-per-(2-carboxyethyl)thio-γ-cyclodextrin-Natriumsalz (SGMD),
Reinigen des rohen SGMD durch Durchleiten durch Adsorbenzien, mit anschließender Rekristallisierung.

2. Verfahren gemäß Anspruch 1, **dadurch gekennzeichnet, dass** das organische Lösungsmittel N,N-Dimethylformamid (DMF) ist.

3. Verfahren gemäß Anspruch 1 oder 2, **dadurch gekennzeichnet, dass** das Verhältnis (V/W) von Aceton und γ-Cyclodextrin (SM1) 30:1∼150:1, bevorzugt 35:1∼140:1, 40:1∼130:1, 45:1∼120:1, 50:1∼110:1, 50:1∼100:1 und am meisten bevorzugt 60:1∼100:1 ist.

4. Verfahren gemäß einem der Ansprüche 1 oder 2, **dadurch gekennzeichnet, dass** die Lösungsmittel, die bei der Rekristallisierung von rohem SGMD-1 verwendet werden, N,N-Dimethylformamid, Dimethylsulfoxid (DMSO), Methanol, Ethanol, Isopropanol oder Aceton oder die Mischung von zwei der obigen Lösungsmitteln ist, bevorzugt eine Mischung aus Aceton und DMF, eine Mischung aus Aceton und DMSO oder eine Mischung aus Methanol und DMF oder eine Mischung aus Ethanol und DMF und am meisten bevorzugt eine Mischung aus Aceton und DMF ist.

5. Verfahren gemäß Anspruch 4, **dadurch gekennzeichnet, dass** das Verhältnis (V/V) von Aceton/DMF 1:0,3∼1:2,5, bevorzugt 1:0,4∼1:2,4, 1:0,5∼1:2,3, 1:0,6v1:2,2, 1:0,7∼1:2,1 und am meisten bevorzugt 0,8∼1:2,0 ist.

6. Verfahren gemäß einem der Ansprüche 1 bis 5, **dadurch gekennzeichnet, dass** das molare Verhältnis von SGMD-1 und 3-Mercaptopropionsäure (SM2) 1:8∼1:25, bevorzugt 1:9∼1:24, 1:10∼1:22, 1:11∼1:21 und am meisten bevorzugt 1:12∼1:20 ist.

7. Verfahren gemäß einem der Ansprüche 1 bis 6, **dadurch gekennzeichnet, dass** das molare Verhältnis von SGMD-1 und Natriumhydrid 1:10∼1:50, bevorzugt 1:12∼1:48, 1:15∼1:45, 1:17∼1:42, 1:18∼1:40 und am meisten bevorzugt 1:22∼1:40 ist.

8. Verfahren gemäß einem der Ansprüche 1 bis 7, **dadurch gekennzeichnet, dass** die Lösungsmittel, die bei der Rekristallisierung von rohem SGMD verwendet werden, Ethanol, Wasser, Methanol oder Isopropanol oder eine Mischung aus Wasser und einem, ausgewählt aus der Gruppe, bestehend aus Ethanol, Methanol oder Isopropanol, bevorzugt eine Mischung aus Methanol und Wasser oder eine Mischung aus Ethanol und Wasser sind.

9. Verfahren gemäß einem der Ansprüche 1 bis 8, **dadurch gekennzeichnet, dass** die Adsorbenzien ausgewählt sind aus der Gruppe, bestehend aus Aktivkohle, Silikagel, makroporösem Harz, Aluminiumoxid, Molekularsieben und Zeolith, bevorzugt Aluminiumoxid oder Aktivkohle oder eine Mischung davon, wobei bevorzugt das Aluminiumoxid basisches oder neutrales Aluminiumoxid ist.

10. Verfahren gemäß einem der Ansprüche 1 bis 9, **dadurch gekennzeichnet, dass** das Verhältnis (G/G) von rohem SGMD und Adsorbenzien 1:0,1∼1:2,5, bevorzugt 1:0,1∼1:2,3, 1:0,1∼1:2,1, 1:0,2∼1:2,0 oder 1:0,2∼1:1,8 und am meisten bevorzugt 1:0,2∼1:1,5 ist.

## Revendications

1. Procédé de préparation d'un sel de sodium de 6-per-désoxy-6-per-(2-carboxyéthyl)thio-γ-cyclodextrine, qui comprend les étapes suivantes :
la réaction d'une γ-cyclodextrine (SM1) avec de l'iode en présence de triphénylphosphine dans un solvant organique pour donner un intermédiaire, la 6-per-désoxy-6-iodo-γ-cyclodextrine (SGMD-1) ;
l'ajout d'une solution méthanol de méthoxyde de sodium dans le système réactionnel, puis l'ajout d'acétone sans éliminer les solvants sous pression réduite pour obtenir le produit brut de SGMD-1 sous la forme d'un solide après filtration ;
la purification de la SGMD-1 brute par recristallisation ;
la réaction de l'intermédiaire recristallisé (SGMD-1) ainsi obtenu avec l'acide 3-mercaptopropionique (SM2) dans un milieu basique, par exemple de l'hydrure de sodium, pour obtenir un produit brut de sel de sodium de 6-per-désoxy-6-per-(2-carboxyéthyl)thio-γ-cyclodextrine (SGMD) ;
la purification de la SGMD brute par passage à travers des adsorbants suivie d'une recristallisation.

2. Procédé selon la revendication 1, **caractérisé en ce que** le solvant organique est le N,N-diméthylformamide (DMF).

3. Procédé selon la revendication 1 ou 2, **caractérisé en ce que** le rapport (en volume/poids) entre l'acétone et la γ-cyclodextrine (SM1) est de 30:1 à 150:1, de préférence de 35 : 1 à 140 : 1, de 40 : 1 à 130 : 1, de 45 : 1 à 120 : 1, de 50 : 1 à 110 : 1, de 50 : 1 à 100 : 1, et le plus préférentiellement de 60 : 1 à 100 : 1.

4. Procédé selon l'une quelconque des revendications 1 ou 2, **caractérisé en ce que** les solvants utilisés dans la recristallisation de la SGMD-1 brute sont le N,N-diméthylformamide, le diméthylsulfoxyde (DMSO), le méthanol, l'éthanol, l'isopropanol ou l'acétone ou le mélange des deux solvants ci-dessus, de préférence un mélange d'acétone et de DMF, un mélange d'acétone et de DMSO, ou un mélange de méthanol et de DMF ou un mélange d'éthanol et de DMF, et le plus préférentiellement un mélange d'acétone et de DMF.

5. Procédé selon la revendication 4, **caractérisé en ce que** le rapport (en volume/volume) acétone/DMF est de 1: 0,3 à 1: 2,5, de préférence de 1: 0,4 à 1: 2,4, de 1: 0,5 à 1 : 2,3, 1 : 0,6 à 1 : 2,2, de 1: 0,7 à 1: 2,1, et le plus préférentiellement de 1: 0,8 à 1: 2,0.

6. Procédé selon l'une quelconque des revendications 1 à 5, **caractérisé en ce que** le rapport molaire entre la SGMD-1 et l'acide 3-mercaptopropionique (SM2) est de 1: 8 à 1 : 25, de préférence de 1: 9 à 1 : 24, de 1 : 10 à 1 : 22, de 1: 11 à 1 : 21, et le plus préférentiellement de 1: 12 à 1: 20.

7. Procédé selon l'une quelconque des revendications 1 à 6, **caractérisé en ce que** le rapport molaire entre la SGMD-1 et l'hydrure de sodium est de 1:10 à 1: 50, de préférence de 1 : 12 à 1: 48, de 1: 15 à 1 : 45, de 1 : 17 à 1: 42, de 1: 18 à 1 : 40, et le plus préférentiellement de 1: 22 à 1: 40.

8. Procédé selon l'une quelconque des revendications 1 à 7, **caractérisé en ce que** les solvants utilisés dans la recristallisation de la SGMD brute sont l'éthanol, l'eau, le méthanol ou l'isopropanol ou un mélange d'eau et de l'un sélectionné dans le groupe consistant en l'éthanol, le méthanol ou l'isopropanol, de préférence un mélange de méthanol et d'eau ou un mélange d'éthanol et d'eau.

9. Procédé selon l'une quelconque des revendications 1 à 8, **caractérisé en ce que** les adsorbants sont sélectionnés dans le groupe consistant en un charbon actif, un gel de silice, une résine macroporeuse, un oxyde d'aluminium, les tamis moléculaires et une zéolite, de préférence un oxyde d'aluminium ou un charbon actif ou un mélange de ceux-ci, de préférence l'oxyde d'aluminium est un oxyde d'aluminium basique ou un oxyde d'aluminium neutre.

10. Procédé selon l'une quelconque des revendications 1 à 9, **caractérisé en ce que** le rapport (en poids/poids) entre la SGMD brute et le ou les absorbant(s) est de 1:0,1 à 1: 2,5, de préférence de 1: 0,1 à 1 : 2,3, de 1: 0,1 à 1: 2,1, de 1: 0,2 à 1 : 2,0, ou de 1 : 0,2 à 1 : 1,8, et le plus préférentiellement de 1 : 0,2 à 1 : 1,5.
